(19) Europäisches Patentamt

European Patent Office

Office européen des brevets

(11)  EP 2 405 782 B1

# (12) FASCICULE DE BREVET EUROPEEN

(45) Date de publication et mention
de la délivrance du brevet:
**12.06.2013 Bulletin 2013/24**

(21) Numéro de dépôt: **10710434.1**

(22) Date de dépôt: **12.03.2010**

(51) Int Cl.:
***A61F 13/08*** *(2006.01)*

(86) Numéro de dépôt international:
**PCT/IB2010/000522**

(87) Numéro de publication internationale:
**WO 2011/132011 (27.10.2011 Gazette 2011/43)**

(54) **ARTICLE DE COMPRESSION AVEC EFFET THERAPEUTIQUE ET/OU PHYSIOLOGIQUE CONCU POUR FACILITER L'ENFILAGE AYANT UN NIVEAU DE COMPRESSION ELEVE**

ZUM LEICHTEREN ANLEGEN AUSGEFÜHRTER KOMPRESSIONSGEGENSTAND MIT THERAPEUTISCHER UND/ODER PHYSIOLOGISCHER WIRKUNG MIT HOHEM KOMPRESSIONSNIVEAU

COMPRESSION ITEM WITH A THERAPEUTIC AND/OR PHYSIOLOGICAL EFFECT, DESIGNED TO FACILITATE THE PULLING-ON THEREOF WITH A HIGH LEVEL OF COMPRESSION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **13.03.2009 FR 0951591**

(43) Date de publication de la demande:
**18.01.2012 Bulletin 2012/03**

(73) Titulaire: **SIGVARIS AG**
**9014 St. Gallen (CH)**

(72) Inventeurs:
• **GANZONI, Stephan**
**4103 Bottmingen (CH)**

• **BERTHEAS, Alain**
**42170 Saint Just Saint Rambert (FR)**
• **SCELLES, Hervé**
**42170 Saint Just Saint Rambert (FR)**
• **FAYOLLE, Christophe**
**42600 Precieux (FR)**

(74) Mandataire: **Dupuis, François et al**
**Cabinet Laurent et Charras**
**3 Place de l'Hôtel-de-Ville**
**B.P. 203**
**42005 St. Etienne Cedex 1 (FR)**

(56) Documents cités:
**EP-A- 1 621 164    EP-A- 1 709 947**
**EP-A- 1 878 822    WO-A-2004/106607**
**US-B1- 7 434 423    US-B1- 7 441 419**

## Description

L'invention se rattache au secteur technique des articles de contention et/ou de compression avec effet thérapeutique et/ou physiologique destiné à être porté par des individus dans des situations variées selon les applications sur les membres inférieurs et/ou supérieurs de l'individu.

Plus spécifiquement, l'invention concerne les bas médicaux de compression, dits BMC, apportant un haut niveau de compression. On entend par « bas » de compression / contention l'ensemble des produits assurant cette fonctionnalité compression / contention, et en particulier les chaussettes, les bas jarrets ou mi-bas, les bas auto-fixant, les bas cuisses, collant, les collants conforts, les collants de maternité.

L'invention concerne également le secteur technique des manchons, des coudières, qui sont susceptibles d'être portés par les membres supérieurs de l'individu.

Les articles de compression / contention ainsi identifiés ci-avant se présentent généralement selon un dispositif tubulaire obtenu par intégration d'une trame sur un article tricoté ou tissé sur métier circulaire ou rectiligne, le produit final étant obtenu en une ou plusieurs fois.

## PROBLEMATIQUE A LA BASE DE L'INVENTION

Les articles de compression / contention avec effet thérapeutique et/ou physiologique sont destinés à être enfilés sur les membres inférieurs et/ou supérieurs d'un individu, et la morphologie naturelle de l'individu implique le passage de l'article et son enfilement du pied ou du poignet de forme et de volume différents et supérieur aux parties des membres suivants, jarret ou bras, dont les configurations sont plus homogènes.

Il y a donc une difficulté d'enfilage qui est d'autant plus difficile que le niveau de compression de l'article est élevée en fonction des effets thérapeutiques et/ou physiologiques recherchés. Les niveaux de compression varient de 1 à 4, pour le référentiel français le niveau 4 étant le plus élevé en effet de compression. Le retrait de l'article pose aussi certaines difficultés.

Par exemple, dans l'application des bas médicaux de compression appartenant aux fortes classes de compression (classes 3, 20 à 36 mmHg, et 4 supérieur à 36 mmHg), cette difficulté d'enfilage est une cause majeure de non observance du traitement par le patient.

Une enquête menée en 2007 par J.J. GUEX identifiée *« La compression médicale classe 3 (20-36mmHg) vue par les médecins et les patients en France »* a démontré que pour 70 % des médecins interrogés, la principale cause de non observance du traitement est la difficulté d'enfilage. Cette enquête, dont le Demandeur est co-auteur, est publiée dans Phlébologie 2009, 62,1.

Il faut considérer et tenir compte que la cible des individus concernés (les porteurs de fortes compressions) peut connaître encore davantage de difficultés d'enfilage liées à certaines spécificités : population plutôt âgée voire très âgée, femmes enceintes, personnes pour lesquelles la dextérité, la force physique ou la mobilité peuvent être limitées.

En outre, la situation de la position physique de l'individu lors de l'enfilage de l'article est susceptible de constituer un paramètre important dans cette difficulté d'enfilage. La position assise, couchée ou debout en appui sur un support, la hauteur habituelle des appuis sont aussi à considérer.

Les tests qui ont été effectués par le Demandeur mettent en évidence la complexité de l'enfilage qui ne se résume pas à une simple traction sur le bas ou à un enfilage par retournement aussi simple que celui décrit dans les recommandations d'enfilage apparaissant sur les notices.

En pratique, on a constaté que différents temps de mise en place sont nécessaires et sont le fait de plusieurs paramètres patients tels que la flexion dorsale en position assise, la flexion de la cheville, en particulier pour les personnes à mobilité restreinte.

Il en résulte en pratique que certains patients devant porter des bas de compression médicale classe 3 se reportent sur un produit de classe 2. Ainsi, la posologie en vue d'un effet thérapeutique précis n'est pas respectée. Certains médecins anticipent même la réaction de leur patient en prescrivant un bas de classe inférieure à ce qui devrait être porté pour assurer une compression minimum.

Il arrive aussi que des patients enfilent deux bas successifs de compression moindre de classes 1 et 2 pensant aboutir au même résultat de compression d'un bas de classe 3, ce qui n'est évidemment pas l'équivalent.

Cette problématique est reconnue par tous, patients, médecins, prescripteurs et fabricants.

## ART ANTERIEUR

Nombreuses sont les tentatives pour remédier à cette problématique, qui concerne aussi bien pour les fabricants à améliorer leurs produits et accessoirement à apporter des moyens complémentaires et indépendants d'aide à l'enfilage.

Sur le premier aspect, certains fabricants ont tenté d'apporter des solutions. La société italienne CIZETA propose ainsi des bas médicaux de contention / compression « avec un tricotage pied large » pour un meilleur enfilage.

Il s'agit donc d'une solution technique qui concerne une partie du bas répondant partiellement à la problématique.

La société française INNOTHERA propose le concept « FOOT IMPROVED PROFILE » pour faciliter l'enfilage, avec un nouveau profil de pression spécifiquement adapté à la zone du pied, le reste du bas en particulier sa partie montante dite « tige » restant classique. Cette société INNOTHERA est aussi titulaire du brevet FR 2885035, US 2006/247566 décrivant un nouveau profil de compression dans la zone allant du cou-de-pied aux malléoles. Il s'agit de réduire drastiquement la pression au niveau du pied et d'apporter une progressivité du profil de pression entre le pied et la cheville. Mais la facilité d'enfilage n'est traitée que partiellement tant que la partie cheville voire même l'intégralité du dispositif n'est pas abordée.

En termes de brevets, le Demandeur a aussi considéré et pris connaissance de manière large des documents suivants :

FR 2853525, FR 2789301, PCT WO 00/01332, WO 01/64152, WO 2008/053159, FR 2804695, FR 2888855, FR 2896808, US 2008/0171959, FR 2703243, FR 2852509, EP 927014, WO 2007/113430, FR 2654925.

Ce dernier document décrit un bas de contention / compression à usage médical incluant un fil élastique en élasthanne de 310 dTex noyé dans un enrobage de fils multibrins.

Aucun de ces documents, ni le brevet FR 2654925, ne répondent néanmoins au problème posé de l'enfilage.

On connaît aussi par le brevet WO 2004/106607 un bas dit thérapeutique avec un titrage de fil épais mais n'ayant aucune particularité et spécificité pour permettre et faciliter son enfilage par élargissement sélectif.

Le brevet EP 1 878 822 décrit une méthode de tricotage permettant de donner un effet visuel de moelleux à un article chaussant de compression mais non directement en rapport avec la problématique de l'invention.

Ce brevet antérieur est connu du Demandeur, certains des inventeurs étant ceux de la présente demande et pouvant parfaitement en différencier les caractéristiques. Ce brevet n'a pas pour fonction d'élargir le bas.

Les brevets US 7 441 419, US 7 434 423, EP 1 709 947, EP 1 621 164 sont également connus mais ne répondent pas à la problématique de l'invention pour faciliter l'enfilement des bas.

Concernant les aides à l'enfilage, ils sont établis pour aider l'utilisateur et/ou un tiers assistant, pour enfiler et/ou faire enfiler les bas. Différents fabricants proposent de tels moyens identifiés sous les références « CHAUSSEBAS » pour la société MEDI, « L'ENFILE-BAS » pour la société COGNON MORIN. Certains dispositifs aident aussi pour le retrait du bas comme celui identifié sous la référence « MEDIVEN SAMBA » de la société MEDI. En pratique, ces aides rendent plus complexe l'enfilage, et peuvent être encombrantes et coûteuses.

Au titre de l'art antérieur, le Demandeur précise qu'avant 1989, il fabriquait déjà des bas médicaux de compression ayant des titrages de 1.769 dTex et de 2.373 dTex. Ces produits de première génération sont caractérisés par une très forte compression qui s'effectue principalement dans la partie cheville avec la compression la plus forte et qui diminue progressivement jusqu'en haut de la cuisse. Ces produits sont très épais, peu esthétiques et difficiles à enfiler du fait de leur rigidité. Ils présentent un dimensionnement identique à plat à ceux des bas de même taille.

Ainsi, le Demandeur a pu faire le constat que la problématique de l'enfilage de l'article de compression / contention et du bas médical de compression demeure, et que les solutions actuellement sur le marché à l'initiative d'une pluralité de fabricants ne sont pas satisfaisantes.

## BUTS RECHERCHES

Un premier but recherché était la conception d'un article de compression / contention du type bas médical de compression par exemple apportant un haut niveau de compression / contention supérieur à 20 mmHg associé à l'efficacité thérapeutique et physiologique, tout en facilitant l'enfilage de l'article sur la totalité du membre récepteur.

Un autre but recherché était la conception d'un article de compression / contention qui, tout en répondant à la problématique, ne génère pas l'adjonction de composants indépendants complexes surenchérissant les coûts de fabrication.

Un autre but recherché était la conception d'un article de compression / contention qui assure un haut degré d'observance et d'autonomie du patient sans avoir recours à un tiers ou à une aide de l'enfilage.

Un autre but recherché était la conception d'un article de compression / contention qui puisse être enfilé aisément, de sorte que le patient utilisateur puisse porter l'article selon le degré de compression / contention le plus adapté à son besoin, et donc sans être obligé ou quasiment obligé de choisir un article ayant des propriétés de compression / contention de valeur moindre mais plus facile à enfiler.

Tous ces buts recherchés et objectifs mis en avant par le Demandeur lui ont permis de développer une réflexion globale en maîtrisant les paramètres, tout cela pour aboutir à une solution technique inattendue répondant en pratique à la problématique ainsi qu'il sera précisé et exposé par la suite.

Il est ainsi nécessaire pour la compréhension de l'invention, de présenter et de rappeler certaines dispositions connues dans la fabrication des bas médicaux de compression à titre d'exemple.

**a) Rappel de la construction classique d'un bas médical de** contention / compression.

1 - Les bas médicaux de contention / compression à un haut niveau de compression utilisent une trame constituée d'une âme en élastomère et plus spécifiquement en élasthanne ayant un titrage utilisé pour la classe 3 jusqu'à 620 dTex. Cette trame est guipée en respectant le cahier des charges réglementaire qui impose notamment un guipage conventionnel en double hélice et un titrage minimum. Ce guipage permet de contrôler l'extensibilité du fil de trame et de le protéger contre les contraintes externes, l'abrasion notamment.

Le tableau 1 illustre ci-après pour les bas médicaux de contention /compression de différents fabricants le titrage des âmes en élastomère des fils de trame en classe 3. Les valeurs vont de 310 à 640 dTex. Les dimensions d'application sont celles indiquées par les fabricants. Les dimensions à plat et les titrages sont évalués au laboratoire d'essai du Demandeur.

### TABLEAU 1

| Analyse produits existant sur le marché. Dimensions à plat et titrage des âmes (élastomères) utilisés sur les BMC classe 3 | | | | | | | |
|---|---|---|---|---|---|---|---|
| Mesure à plat | Taille | Cheville (cm) | Mollet (cm) | Cuisse | Titrage Elastomère | Périmètre app. mini (cm) | Périmètre app. maxi (cm) |
| Varisma Comfort coton 3 Innothera | T1 court | 7.4 | 11,7/11,8 | 16,7/16,8 | EA 540dTex | | |
| | T2 court | 7,8/7,9 | 11,6/11,9 | 15,4/15,6 | | 21,5 | 23,5 |
| | T3 court | 8.5 | 12,9/13 | 17,9 | | | |
| | T4 court | 9.3 | 13,5/13,8 | 19 | | | |
| | T1 normal | 7,4/7,5 | 12 | 18,1/18,2 | | | |
| | T2 normal | 7,9/8 | 12,9/13 | 18.9 | | | |
| | T3 normal | 8,9/8,8 | 13.5 | 18,8/18,7 | | | |
| Venoflex Elégance H 3 Thuasne | T2N | 7,8 | 12/12,2 | 17,2/17,3 | EA 470dTex | 21 | 24 |
| Venoflex Transparence F 3 | T2N | 8,6/8,7 | 12/12,2 | 16 | | | |
| Venoflex Douceur F 3 | T2 N | 8,3 | 12,5 | 17 | | | |
| Mediven Elegance 30 | TM Court | 7 | 10,2 | 14 | EA 470dTex | 21 | 23 |
| Jobst Caresse | T2 Normal | 7.3 | 10,5 | 17,2 | EA | 20 | 22 |
| Jobst Allure | T2 Normal | 7,3 | 10,5 | 18/18,1 | 395dTex | | |

(suite)

| Analyse produits existant sur le marché. Dimensions à plat et titrage des âmes (élastomères) utilisés sur les BMC classe 3 | | | | | | | |
|---|---|---|---|---|---|---|---|
| Mesure à plat | Taille | Cheville (cm) | Mollet (cm) | Cuisse | Titrage Elastomère | Périmètre app. mini (cm) | Périmètre app. maxi (cm) |
| Radiante 93 Coton Classe 3 Femme | T2 Court | 7,3 | 10,5 | 14,3/14,4 | Gomme 90 (637 dTex) | 22.5 | 24,5 |
| | T3 Court | 8,1 | 11,4 | 14,6/14,7 | | | |
| | T4 Court | 8.6 | 11.6 | 15,2/15,3 | | | |
| | T5 Court | 8.9 | 11,9 | 15,5/15,5 | | | |
| | T1 Moyen | 7.5 | 10,1 | 12.6/13 | | | |
| | T4 Moyen | 8/8,5 | 11,5/11,6 | 15,3/15,5 | | | |
| | T5 Moyen | 8,9/9 | 11,6/11,7 15,8/16 | | | | |
| | T3 Long | 8,3/8,4 | 11,4/11,5 | 14,6/14,7 | | | |
| Radiante 93 Coton Classe 3 Homme | T2 Court | 7,6 | 10,5/10,6 | 14,5 | Gomme 90 (637 dTex) | 22,5 | 24.54 |
| | T 3 Court | 7,9 | 10,9/11 | 14,5/14,6 | | | |
| | T 1 Moyen | 7/7,1 | 10,3/10,4 | 14,1/14,2 | | | |
| | T 3 Moyen | 7.8 | 10,7/10,8 | 15 | | | |
| | T 5 Moyen | 9,1/9,2 | 13,8 | 16/16,2 | | | |
| | T 2 Long | 7,5/7,6 | 10.2 | 14,5/14,6 | | 22.5 | 24,54 |
| | T 3 Long | 8/8,1 | 10.6 | 15.5 | | | |
| | T 4 Long | 8,4/8,5 | 11,3 | 16 | | | |
| | T 5 Long | 9,3/9,4 | 13,5 | 16,8/16,9 | | | |

| Mesure à plat | Taille | Cheville (cm) | Mollet (cm) | Cuisse | Titrage Elastomère | Périmètre app. mini (cm) | Périmètre app. maxi (cm) |
|---|---|---|---|---|---|---|---|
| Venactif Douceur Gibaud | T2 | 7,1/7,2 | 11,9/12 | 15,8/15,9 | EA 285/310 dTex | 20 | 23 |
| Veinostim Caresse Pierre F. | T2N | 8,1/8,1 | 9,4/9,6 | 14,1/14,3 | EA 285/310dTex | 22 | 24 |
| Veinostim Finesse Pierre F. | T2N | 8,1/8,1 | 10,3/10,4 | 15,7/15,6 | | | |
| Veinamitex Labo Pharma | T2N | 7,4 | 11 | 17,5 | EA 395dTex | 20 | 23 |

(suite)

| Mesure à plat | Taille | Cheville (cm) | Mollet (cm) | Cuisse | Titrage Elastomère | Périmètre app. mini (cm) | Périmètre app. maxi (cm) |
|---|---|---|---|---|---|---|---|
| Sigvaris Coton 3 H | M | 8,2 | 13 | 16,9 | | | |
| Sigvaris Microfibre 3 H | M | 8,4 | 13 | 18,5 | EA 620dTex | 22 | 24 |
| Sigvaris Coton 3 F | M | 8,2 | 13 | 16,9 | | | |
| Sigvaris Microfibre 3 F | M | 7,9 | 12 | 17,5 | | | |

La trame est ensuite intégrée dans la structure textile tricotée avec une densité appropriée liée à la hauteur de la maille pour avoir dans la zone concernée le niveau de compression voulu pour une dimension d'application donnée (mises en extension transversale et longitudinale de la structure textile tricotée ou article lorsqu'il est porté).

2 - Le taillage de l'article, en l'espèce un bas médical de contention / compression, permet d'établir un tableau de taillage en fonction de la morphologie du patient avec les trois zones successives b) tour de cheville, c) tour de mollet, m) tour de cuisse. Le tableau ci-après donne des indications de tailles en fonction des dimensions références XS, S, M, L, XL, XXL, correspondant à une normalisation spécifique.

## <u>TABLEAU 2</u>

## SYSTEME DE TAILLAGE CLASSE 3

## FEMME

**Chaussettes - BAF**

| | b | c | m BAF |
|---|---|---|---|
| XS | 17 - 19 | 29 - 35 | 42 - 59 |
| S | 19 - 22 | 32 - 38 | 45 - 62 |
| M | 22 - 24 | 35 - 41 | 48 - 65 |
| L | 24 - 26 | 38 - 44 | 51 - 68 |
| XL | 26 - 29 | 41 - 47 | 54 - 71 |
| XXL | 29 - 32 | 44 - 50 | 57 - 74 |

**Hauteur chaussettes**

| | Normal | Long |
|---|---|---|
| A-G | ≤ 40 cm | ≥ 40 cm |
| Pointures | 36/40 | 37/41 |

**Hauteur BAF**

| | Normal | Long |
|---|---|---|
| A-D | ≤ 75 cm | ≥ 75 cm |
| Pointures | 36/40 | 37/41 |

## HOMME

**Chaussettes - BAF**

|  | b | c | m BAF |
|---|---|---|---|
| S | 19 - 22 | 32 - 39 | 46 - 58 |
| M | 22 - 24 | 35 - 42 | 48 - 62 |
| L | 24 - 26 | 38 - 45 | 54 - 66 |
| XL | 26 - 29 | 41 - 48 | 58 - 70 |
| XXL | 29 - 32 | 44 - 51 | 62 - 74 |

**Hauteur chaussettes**

|  | Normal | Long |
|---|---|---|
| A-G | ≤ 42 cm | ≥ 42 cm |
| Pointures | 40/43 | 43/46 |

**Hauteur BAF**

|  | Normal | Long |
|---|---|---|
| A-D | ≤ 79 cm | ≥ 79 cm |
| Pointures | 40/43 | 43/46 |

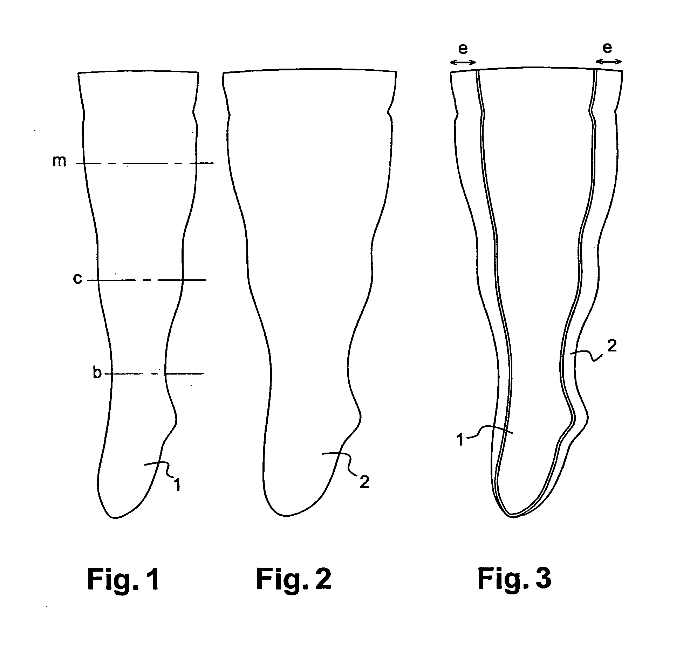

La dimension à plat de l'article est ainsi une résultante de cette construction et correspond au dimensionnel de l'article posé à plat. On a ainsi représenté figure 1 un tel article (1) dans le cas d'un bas médical de contention / compression.

**b) comportement mécanique** des bas médicaux de contention / compression dans le cadre de la mesure de la compression et de la courbe allongement en tenant compte de la norme NF G 30-102. Des analyses ont été effectuées et il y sera fait référence par la suite comparativement à l'article selon l'invention.

**c)** Evaluation du coefficient de friction de textures de bas médicaux chez des sujets sains au niveau du cou-de-pied. Cette étude a été réalisée par le Centre d'Etudes et de Recherche sur le Tégument au CHU de Besançon en France, à l'initiative du Demandeur.

Il ressort de cette étude que la peau du cou-de-pied est fortement hydratée. Ainsi, la problématique liée à des difficultés d'enfilage du fait du frottement avec la matière est réelle et généralisable, même aux personnes qui n'ont pas tendance à transpirer.

**d)** Des travaux sur la glissabilité des matières (coefficient de friction peau /textile) ont été réalisés après avoir déterminé les problématiques liées à la peau du cou-de-pied.

## **INVENTION**

C'est à partir de l'ensemble de ces éléments et données scientifiques que le Demandeur a cherché à concevoir un nouvel article de contention / compression à un niveau de compression élevée.

L'ensemble de ces analyses a conduit le Demandeur à s'orienter vers une construction différente de celles habituellement utilisées dans les constructions classiques, répondant aux conditions de titrage rappelées précédemment.

La solution apportée par le Demandeur apporte des résultats inattendus et répondant spécifiquement à la problématique posée en assurant un enfilage aisé limitant la contrainte et l'assistance extérieure de l'article confectionné.

Cette solution implique une sélection particulière dans la conception d'un fil de trame guipé avec une âme en élastomère et élasthanne en particulier qui entraine après une fabrication classique de l'article dans les conditions usuelles une configuration de l'article confectionné différente et parfaitement identifiable par rapport à un article de contention / compression de même classification.

Ainsi, selon l'invention, l'article de contention / compression ayant un niveau de compression élevé supérieur à 20mmHg du type tricoté comprenant un fil de trame guipé en double hélice avec une âme centrale en élastomère et notamment en élasthanne, tricoté sur l'ensemble de l'article confectionné, est remarquable en ce que le titrage de la dite âme est compris entre 750 et 1.700 dTex, en permettant l'obtention pour une même classification un article de dimensions supérieures à un article comparable de même catégorie, et en ce que les caractéristiques d'allongement dimensionnel de l'article sur toute sa surface mettent en oeuvre la formule :

$$\frac{Titre}{\sqrt{Comp.moy}} \geq K * \frac{P\acute{e}rim.app.\min}{L\arg.\grave{a}.plat}$$

où le facteur K est égal à 55 et exprimé en $\dfrac{dTex}{\sqrt{mmHg}}$

et en ce que la déformation du dit article présente un coefficient d'allongement en situation et pour les dimensions minimales d'applications inférieur à 25%,
et en ce que la formule

$$\frac{P\acute{e}rim.app.\min}{L\arg.\grave{a}.plat}$$

représente la caractéristique dimensionnelle de l'invention,
et en ce que la formule

$$\frac{Titre}{\sqrt{Comp.moy}}$$

représente le rapport entre le titre de l'âme constitutive du fil de trame et le niveau de compression,
et en ce que la combinaison des deux formules et le coefficient K, facteur multiplicatif, définit la caractéristique sélective du dit article de contention /compression.

Selon une autre caractéristique, l'âme en élastomère et notamment en élasthanne constitutive du fil de trame présente un titrage de l'ordre de 900 à 1.000 dTex.

Ces caractéristiques et d'autres encore ressortiront bien de la suite de la description.

Pour fixer l'objet de l'invention illustrée d'une manière non limitative aux figures des dessins où :

- la figure 1 est une vue à plat selon l'art antérieur d'un article de contention / compression du type bas médical selon une taille donnée.
- la figure 2 est une vue à plat d'un bas médical de contention / compression réalisé selon l'invention et ayant la même taille que celui de la figure 1.
- la figure 3 est une vue à plat selon les figures 1 et 2 représentant les deux bas des figures 1 et 2 en superposition et faisant apparaître leur différence de dimensions.
- la figure 4 illustre le portage des deux bas selon l'art antérieur et selon l'invention.
- la figure 5 illustre l'enfilage des bas selon l'art antérieur et selon l'invention au niveau du pied.
- la figure 6 illustre l'enfilage des bas selon l'art antérieur et selon l'invention au niveau du mollet.
- la figure 7 illustre l'enfilage des bas selon l'art antérieur et selon l'invention au niveau de la cuisse.
- Le tableau 5 est un comparatif entre un produit classique et le bas selon l'invention.

Afin de rendre plus concret l'objet de l'invention, on le décrit maintenant d'une manière non limitative illustrée aux figures des dessins.

L'article de contention / compression concerné par l'invention se rapporte à tous les exemples cités de manière non limitative dans le préambule de la description. L'invention est décrite ci-après pour son application non limitative à un bas de compression / contention à titre d'exemple, mais l'originalité de l'invention se rapporte à la définition générale initiale de l'article.

Pour la bonne compréhension de l'invention et de ses résultats, il sera fait référence à des tableaux comparatifs en considérant bien entendu que les produits sont fabriqués dans les mêmes conditions, sont de même catégorie, tableaux incluant également les caractéristiques spécifiques au produit de l'invention qui lui confèrent son originalité et sa spécificité pour répondre à la problématique de l'enfilage par le patient.

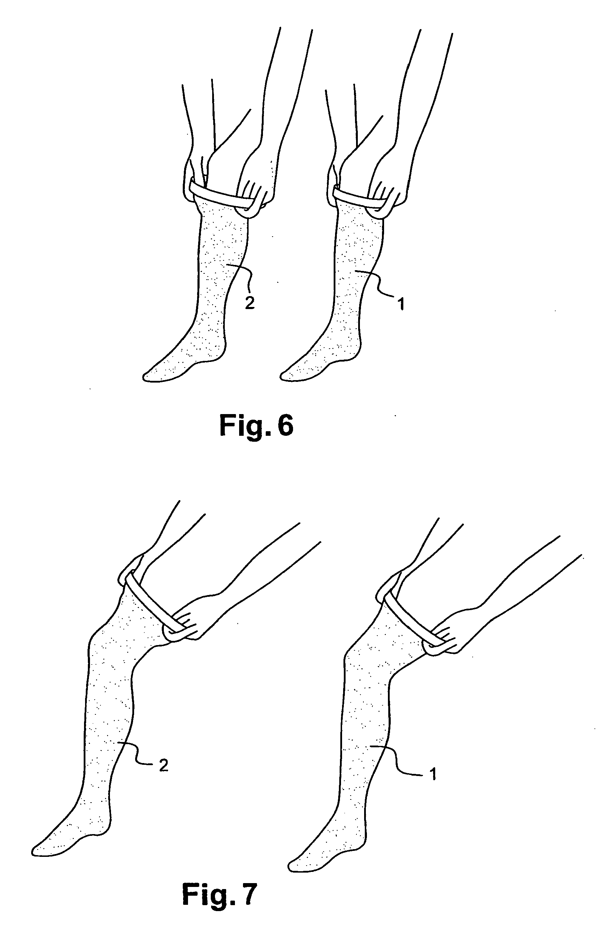

En se référant aux figures 1 à 7, l'article du type bas médical de contention / compression issu de l'art antérieur est référencé par (1) et celui objet de l'invention par (2). Ils sont fabriqués à partir des mêmes matériaux, des mêmes structures textiles, utilisent un fil de trame enroulé en hélice ou double hélice sur la totalité du corps de l'article, le fil de trame étant guipé et réalisé en élastomère et plus spécifiquement en élasthanne. L'article est tricoté.

La différence selon l'invention réside dans le fait que le fil de trame dans le cas du bas (1) présente un titrage choisi dans la fourchette connue de 310 à 640 dTex tel qu'identifié dans le tableau 1, alors que selon l'invention, le fil de trame est guipé et comprend une âme en élastomère et en élasthanne en particulier, la dite âme présentant un titrage beaucoup plus important que le titrage maximum connu de 640 dTex en étant donc choisi et sélectionné avec un titrage de 750 à 1.700 dTex soit une augmentation minimum de 750/640 = 17 %. Dans le cadre d'une optimisation de l'invention, le titrage de l'âme du fil de trame se situe entre 900 et 1.000 dTex et de préférence 940 dTex, soit une augmentation de 940/640 d'environ 50 %.

Cette sélection particulière du titrage de l'âme du fil de trame en élasthanne très au-delà des usages et normes combiné à d'autre caractéristiques ci-après exposées permet d'aboutir à la réalisation d'un article de contention / compression répondant à la problématique et apparaissant visuellement surdimensionné (figure 3) par rapport à un bas médical de contention / compression classique tout en étant d'une même catégorie de taille. Les figures 1 à 3 sont ainsi très explicites dans la représentation des bas à plat avant portage. On a identifié par (e) l'écartement dimensionné en partie supérieure.

Cette caractéristique particulière de titrage est combinée avec l'application de la formule suivante.

$$\frac{Titre}{\sqrt{Comp.moy}} \geq K * \frac{P\acute{e}rim.app.\min}{L\arg.\grave{a}.plat}$$

Et $750 \leq$ Titre $\leq 1700$ dTex

$$facteur\ K = 55\ (\frac{dTex}{\sqrt{mmHg}})$$

Ce facteur K est un facteur multiplicatif permettant de mettre en relation les deux parties de formules, droite et gauche, et prenant en compte les unités de mesures. Ce facteur K est établi à la valeur de 55.

Voici les unités utilisées dans cette formule :

→ Titre : Masse linéique du fil d'élasthanne constituant l'âme contenue dans le fil de trame en dtex, et par extension de l'élastomère utilisé.

→ Com. Moy : Compression moyenne dans la classe de compression concernée en mmHg. (Exemple, pour une Classe 3 française, Comp. Moy. = 28 mmHg). Pour une classe 4 (>36mmHg), en l'absence de précision d'une plage de compression de la part du fabricant, la compression moyenne est déterminée à partir d'articles commercialisés, selon la méthode de référence

→ Larg. à plat : Largeur à plat du produit mesurée à la cheville en cm (selon la taille). Par extension, concerne la largeur au niveau du poignet pour un manchon positionné sur le membre supérieur.

→ Périm.app.min : Périmètre d'application minimum à la cheville en cm (suivant la taille concernée). Par extension, concerne le périmètre d'application minimum au niveau du poignet pour un manchon positionné sur le membre supérieur. Il est précisé que le périmètre moyen d'application n'a pas été retenu dans la dite formule car les plages minima et maxima n'ont pas toutes la même amplitude selon la taille et /ou le fabricant.

La partie gauche de la formule prend en compte les composantes élasto-mécaniques, avec comme spécificité pour l'invention un titre particulièrement élevé pour la compression visée. Ce rapport $\frac{Titre}{\sqrt{Comp.moy}}$ apparaît dans le tableau dans le cadre de l'invention (tableau 3 - Suite - SIGVARIS EXPERT) particulièrement élevé, le titre utilisé pour l'âme constitutive de l'âme du fil de trame étant important compte tenu du niveau de compression souhaité.

La partie droite de la formule prend en compte les composantes dimensionnelles, avec comme spécificité pour l'invention un dimensionnel à plat particulièrement important au regard des dimensions d'application. Cette partie de formule $\frac{P\acute{e}rim.app.\min}{L\arg.\grave{a}.plat}$ représente la caractéristique dimensionnelle de l'invention. Le rapport est dans le cas de l'invention (tableau 3 suite - SIGVARIS EXPERT) particulièrement faible, la largeur à plat du bas étant élevée par rapport à la circonférence minimum d'application. Ce rapport est en liaison avec le coefficient d'allongement de l'article en situation, aux dimensions minimas d'application, inférieur à 25 %.

La formule globale avec le choix du coefficient K est caractéristique de l'invention puisqu'elle indique par leur mise en relation que les deux parties de formules droite et gauche sont symptomatiques de la construction retenue. Les deux parties de formules varient de façon opposée car le titrage de l'âme est particulièrement élevé pour les compressions visées (formule gauche avec résultat particulièrement élevé) et le dimensionnel de l'article est élargi (formule de droite avec résultat particulièrement faible). Cette sélection des deux parties définies en situation d'inversion et le choix du coefficient K permettent l'obtention des propriétés de l'invention et ce en combinaison avec le coefficient d'allongement inférieur à 25 %.

Les termes et la combinaison des éléments constitutifs de cette formule viennent à l'encontre des connaissances actuelles appliquées sur les produits du marché.

Le tableau 3-3 (suite) donne une illustration complémentaire avec deux constructions habituelles permettant d'obtenir le même niveau de compression.

- Cas de l'article dans lequel la jambe de droite a un faible résultat (peu d'écart entre dimensionnel article et dimension d'application). La formule de gauche l'est également ce qui signifie que le titrage n'est pas anormalement élevé compte tenu du niveau de compression visé. Exemple : VENOFLEX Douceur.

- A l'inverse, cas de l'article pour lequel la formule de gauche est élevée, le titrage étant plutôt élevé par rapport à la compression obtenue : la formule de droite l'est également, ce qui signifie que les dimensions à plat de l'article sont petites. Exemple : RADIANTE COTON.

Deux exemples sont donnés aussi à partir d'un produit commercialisé par le demandeur SIGVARIS COTON 3 HOMME et un des produits suivant l'invention SIGVARIS EXPERT pour l'explication de l'application de la formule. Il est considéré la classe 3 des bas médicaux de compression et la valeur moyenne de compression en est de 28.

### Exemple SIGVARIS COTON :

La formule devient :

$$\frac{650}{\sqrt{28}} \geq 55 * \frac{22}{8.2}$$ soit 117,11 inférieur à 147,56 pas applicable

### Exemple SIGVARIS INVENTION:

La formule devient :

$$\frac{940}{\sqrt{28}} \geq 55 * \frac{22}{8}$$ soit 177,64 supérieur à 151,25 applicable

Cette dernière valeur valide l'application de l'invention en fonction des caractéristiques de titrage sélectionné.

Le tableau T3 donne par ailleurs, pour différents produits sur le marché, et celui objet de l'invention identifié SIGVARIS EXPERT, les valeurs en application.

## TABLEAU 3

| PRODUIT | DIMENSIONS D'APPLICATION | | TITRAGE AME | LARGEUR A PLAT |
|---|---|---|---|---|
| | MINI | MAXI | | |
| Varisma comfort coton3 | 21,5 | 23,5 | 570/620 | 7,9 |
| Radiante coton Hom | 22,5 | 24,5 | 637 | 7,7 |
| Radiante coton Fem | 22,5 | 24,5 | 637 | 7,7 |
| Mediven elegance | 21 | 23 | 395/470 | 6,9 |
| **Sigvaris Expert** | **22** | **24** | **940** | **9** |
| Veinostim caresse (PF) | 22 | 24 | 285/310 | 8 |
| Veinostim finesse (PF) | 22 | 24 | 285/310 | 8 |
| Venactif Douceur | 20 | 23 | 285/310 | 7,2 |
| Venoflex Douceur | 21 | 24 | 470 | 8,4 |
| Venoflex Elegance Hom | 21 | 24 | 470 | 7,9 |

| Venoflex Transparence | 21 | 24 | 470 | 8,7 |
|---|---|---|---|---|
| Jobst Caresse | 20 | 22 | 395/475 | 7,3 |
| Jobst Allure | 20 | 22 | 395/475 | 7,3 |
| Sigvaris micro 3 Fem | 22 | 24 | 475 | 7,9 |
| Sigvaris coton 3 Fem | 22 | 24 | 620 | 8,2 |
| Sigvaris micro 3 Hom | 22 | 24 | 620 | 8,4 |
| Sigvaris coton 3 Hom | 22 | 24 | 620 | 8,2 |

## TABLEAU 3 (suite)

| PRODUIT | Formule de gauche | Formule de droite | Vérification |
|---|---|---|---|
| Varisma comfort coton3 | 107,72 | 149,68 | NO |
| Radiante coton Hom | 120,38 | 160,71 | NO |
| Radiante coton Fem | 120,38 | 160,71 | NO |
| Mediven elegance | 88,82 | 167,39 | NO |
| **Sigvaris Expert** | **177,64** | **134,44** | **YES** |
| Veinostim caresse (PF) | 53,86 | 151,25 | NO |
| Veinostim finesse (PF) | 53,86 | 151,25 | NO |
| Venactif Douceur | 53,86 | 152,78 | NO |
| Venoflex Douceur | 88,82 | 137,5 | NO |
| Venoflex Elegance Hom | 88,82 | 146,20 | NO |
| Venoflex Transparence | 88,82 | 132,76 | NO |
| Jobst Caresse | 74,65 | 150,68 | NO |
| Jobst Allure | 74,65 | 150,68 | NO |
| Sigvaris micro 3 Fem | 89,77 | 153,16 | NO |
| Sigvaris coton 3 Fem | 117,17 | 147,566 | NO |

| Sigvaris micro 3 Hom | 117,176 | 144,05 | NO |
| Sigvaris coton 3 Hom | 117,17 | 147,56 | NO |

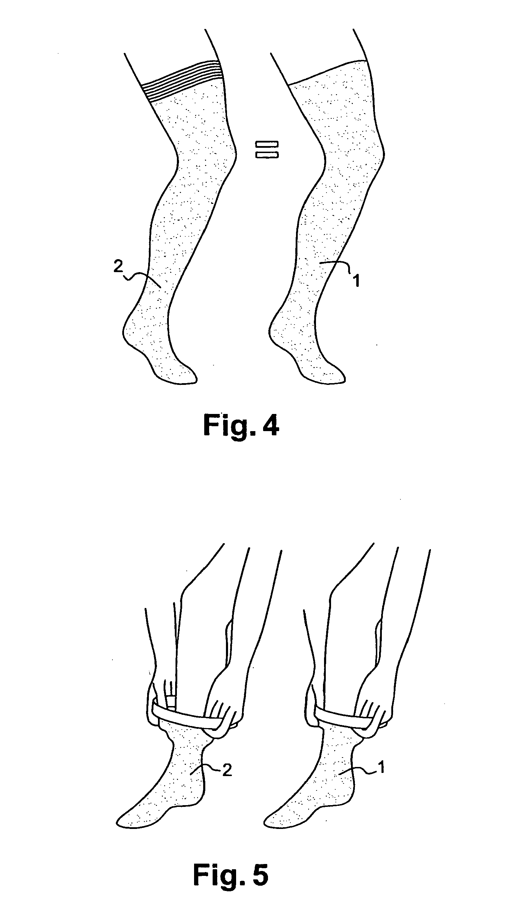

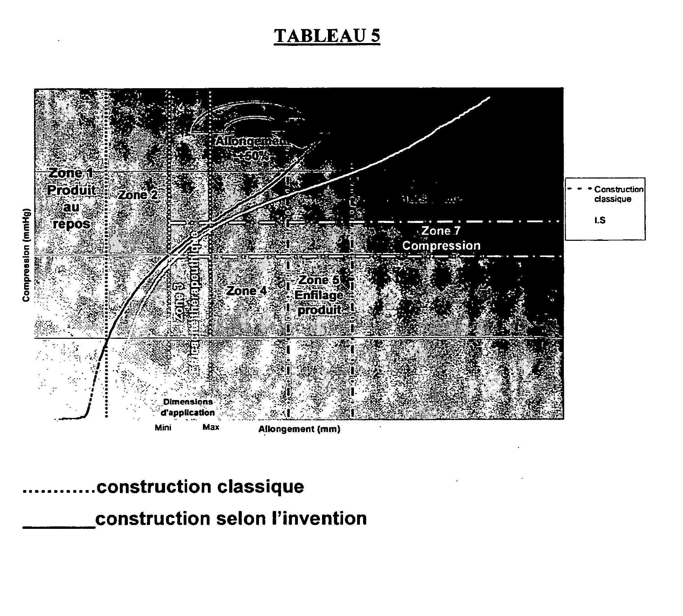

En situation de portage représenté figure 4, la présentation du bas sur la jambe est la même et aucune différence n'apparaît.

On a représenté aux figures 4, 5 et 6 les différents stades d'enfilage des bas (1) art antérieur et (2) invention, montrant bien l'élargissement ainsi sur la totalité du bas de l'invention à des endroits identiques par rapport au bas classique, facilitant ainsi l'enfilage.

L'élargissement moyen, correspondant à la déformation moyenne de l'article, sur toute sa longueur, entre ses dimensions à plat et les dimensions d'application minimales, et répond à la formule suivante :

$$\% \text{ allongement moyen} < 25\%$$

L'élargissement moyen apparaît dans ce tableau T4 suivant la formule précitée avec les produits sur le marché et celui, selon l'invention, identifié par la référence SIGVARIS EXPERT (Homme - Femme).

## TABLEAU 4

## DIMENSIONNEL ELARGI DE L'INVENTION IS
## (exemple applicatif EXPERT pour des BMC de classe 3)

| PRODUIT | Dim. appl. Cheville mini (cm) | circonf. à plat (cm) | % chev | Dim. appl. Mollet mini (cm) | circonf. à plat (cm) | % mollet |
|---|---|---|---|---|---|---|
| Varisma comfort coton3 | 21,5 | 15,8 | 36 | / | 26 | / |
| Radiante coton Hom | 22,5 | 15,4 | 46 | / | 20,4 | / |
| Radiante coton Fem | 22,5 | 15,4 | 46 | 29 | 21 | 38 |
| Mediven elegance | 21 | 13,8 | 52 | 32 | 20,4 | 57 |
| Sigvaris Expert Fem | 22 | 18,2 | 21 | 35 | 28,2 | 24 |
| Sigvaris Expert Hom | 22 | 18,2 | 21 | 35 | 28,4 | 23 |
| Veinostim caresse (PF) | 22 | 16 | 38 | 35 | 19,2 | 82 |
| Veinostim finesse (PF) | 22 | 16 | 38 | 35 | 20,8 | 68 |
| Venactif Douceur | 20 | 14,4 | 39 | 31 | 24 | 29 |
| Venoflex Douceur | 21 | 16,8 | 25 | / | 25 | / |
| Venoflex Elegance Hom | 21 | 15,8 | 33 | 32 | 24,4 | 31 |
| Venoflex Transparence | 21 | 17,4 | 21 | / | 24,4 | / |
| Jobst Caresse | 20 | 14,6 | 37 | 31,5 | 21 | 50 |
| Jobst Allure | 20 | 14,6 | 37 | 31,5 | 21 | 50 |
| VEINAMITEX | 20 | 14,8 | 35 | 32 | 22 | 45 |
| Sigvaris micro 3 Fem | 22 | 15,8 | 39 | 35 | 24 | 46 |
| Sigvaris coton 3 Fem | 22 | 16,4 | 34 | 35 | 26 | 35 |
| Sigvaris micro 3 Hom | 22 | 16,8 | 31 | 35 | 26 | 35 |
| Sigvaris coton 3 Hom | 22 | 16,4 | 34 | 35 | 26 | 35 |

## TABLEAU 4 (SUITE)

| PRODUIT | Dim. applic. Cuisse mini (cm) | circonf. à plat (cm) | % cuisse | Moyenne chev - cuisse | Moyenne globale |
|---|---|---|---|---|---|
| Varisma comfort coton3 | 50 | 37,8 | 32 | 34 | / |
| Radiante coton Hom | 47 | 29,1 | 62 | 54 | / |
| Radiante coton Fem | 47 | 28,7 | 64 | 55 | 49 |
| Mediven elegance | 48 | 28 | 71 | 62 | 60 |
| **Sigvaris Expert Fem** | 48 | 40,4 | 19 | **20** | **21** |
| **Sigvaris Expert Hom** | 50 | 40,4 | 24 | **22** | **23** |
| Veinostim caresse (PF) | 48 | 28,4 | 69 | 53 | 63 |
| Veinostim finesse (PF) | 48 | 31,3 | 53 | 45 | 53 |
| Venactif Douceur | 45 | 31,7 | 42 | 40 | 37 |
| Venoflex Douceur | 49 | 34 | 44 | 35 | / |
| Venoflex Elegance Hom | 50 | 34,5 | 45 | 39 | 36 |
| Venoflex Transparence | 49 | 32 | 53 | 37 | / |
| Jobst Caresse | 45 | 34,4 | 31 | 34 | 39 |
| Jobst Allure | 45 | 36,1 | 25 | 31 | 37 |
| VEINAMITEX | 53 | 35 | 51 | 43 | 44 |
| Sigvaris micro 3 Fem | 48 | 33,8 | 42 | 41 | 42 |
| Sigvaris coton 3 Fem | 48 | 37 | 30 | 32 | 33 |
| Sigvaris micro 3 Hom | 48 | 33,8 | 42 | 36 | 36 |
| Sigvaris coton 3 Hom | 48 | 27,4 | 75 | 55 | 48 |

La déformation subie par l'article entre le repos et les dimensions d'application minimales (se référer au tableau de taillage du fabricant) est caractéristique pour l'invention objet de la demande, et en ce que sur tout l'article, cette déformation est réduite le plus possible du fait de l'élargissement de tout le dispositif et est inférieure à 25%. Cette déformation peut être mesurée soit en deux points extrêmes de l'article (cheville et cuisse pour un bas auto-fixant ou un collant par exemple), ou en 3 points si les données du fabricant sont disponibles (exemple : cheville, mollet et cuisse).

Les autres produits "classiques" ont un taux d'allongement > 30%.

L'âme caractéristique importante de l'invention est celle de l'âme en élastomère notamment en élasthanne à module souple ou élastodiène (gomme naturelle).

Le fil de trame est dans le cas d'un BMC guipé selon le procédé de guipage conventionnel en double hélice (double couverture).

Sur d'autres dispositifs, le guipage peut être conventionnel en simple ou double couverture autour de l'âme centrale, en assemblage air (entremêlement de fils) ou par points de chaînette.

Le fil de trame comporte une ou plusieurs âmes élastiques, pouvant être de natures différentes. Le titrage mentionné dans l'invention correspond au titrage global de celles-ci.

Pour en faciliter la compréhension, il y a lieu de se référer au tableau T5 ci-après qui est un comparatif entre le produit (1) classique et le bas (2) selon l'invention. Le graphe présente le rapport de compression de l'article par rapport à son allongement en identifiant 7 zones : Z1, Z2, Z3, Z4, Z5, Z6 et Z7.

Tableau 5

Le pied de courbe est caractéristique du dimensionnel des articles.

- La zone Z1 : les produits sont au repos et les dimensions à plat sont constantes.

- La zone Z2 correspond à l'étirement du produit pour arriver aux dimensions d'applications.
- La zone Z3 correspond aux dimensions d'application (mini et maxi) avec le niveau d'efficacité thérapeutique recherché.
- La zone Z4 montre l'étirement complémentaire du produit.
- La zone Z5 correspond à la zone d'enfilage de l'article avec un allongement de 50 % de l'article. On distingue parfaitement la différenciation des courbes des bas 1 et 2. Ainsi le choix particulier de l'élastomère de type élasthanne à module souple ou élastodiène contribue à cet effet.
- La zone Z6 sort du domaine d'utilisation et est laissée pour montrer le comportement mécanique.
- La zone Z7 horizontale est la zone de compression thérapeutique. L'intersection entre les zones Z3 et Z7 donne l'efficacité thérapeutique recherchée.

Ainsi, les caractéristiques de l'âme de la trame selon l'invention permettent pour faciliter l'enfilage de l'article d'avoir une augmentation de compression modérée par une déformation supplémentaire de 50 % de la zone Z5 par rapport à une construction classique.

On observe aussi à partir du graphe une zone Z6 décalée par rapport aux articles de contention / compression classique, cette zone n'apportant pas d'effet particulier mais étant représentative de la courbe dans son ensemble.

Cette étude comparative a été établie sur un bas de taille Médium représentatif car correspondant à 37 % de l'ensemble des ventes toutes tailles confondues hommes/femmes.

Des essais et tests ont été réalisés comparativement et ont donc permis de valider le choix technique inattendu le Demandeur en particulier par l'utilisation d'un titrage d'élastomère ou d'élasthanne pour l'âme de la trame anormalement élevé pour le niveau de compression. Cela concerne en particulier la zone Z1 pour le dimensionnel à plat élargi en Z3 pour l'efficacité thérapeutique.

Une étude a été développée à partir de l'article obtenu selon l'invention dans le cadre d'une expérimentation en relation avec le Docteur Eric LEFLOCH. Cette étude portait sur les conditions d'enfilage de l'article réalisé sous forme de bas à partir de porteurs usuels de bas médicaux de contention / compression et de non-porteurs de bas médicaux de contention_/ compression Ce test d'enfilage a ainsi été effectué sur 592 patients, 395 porteurs usuels de bas médicaux de contention / compression et 197 non-porteurs de bas médicaux de contention / compression. Les appréciations ont ensuite été recueillies pour connaître les évaluations sur cet enfilage par les différents porteurs. Il en est résulté que pour les porteurs habituels 66% ont considéré que le bas réalisé selon l'invention était facile ou très facile à enfiler. Ces valeurs sont particulièrement élevées compte tenu de la problématique d'enfilage associée à cette famille de produits de classe de compression élevée. Par ailleurs, 19% considéraient qu'il était d'enfilage normal et 15% difficile ou très difficile à enfiler, sachant que cela ne signifie pas pour autant que l'enfilage est plus difficile qu'avec le produit habituel. Pour les porteurs n'utilisant pas des bas médicaux de contention / compression. 58% ont considéré que le bas selon l'invention était facile ou très facile pour son enfilage. Ces valeurs sont d'autant plus élevées que ces personnes enfilaient pour la première fois un bas médical de compression. Par ailleurs, 21% considéraient un enfilage normal, et 21% considéraient l'enfilage difficile ou très difficile. Cette étude confirme une amélioration substantielle du processus d'enfilage et du sentiment d'aisance pour assurer cet enfilage. Cette étude confirme donc l'intérêt pratique de l'invention.

De manière plus générale les articles de compression / contention mettent en oeuvre la sélection particulière du titrage de l'âme du fil de trame répondant aux buts recherchés et visés. L'intérêt de l'invention réside aussi dans le fait que l'effet donné par la sélection particulière du titrage de l'âme constitutive de la trame sélectionnée se transpose dans la totalité du corps de l'article, puisque le fil de trame est enroulé en hélice sur toute la constitution de l'article. L'article en totalité est donc concerné et sa facilité d'enfilage ou de retrait s'apprécie sur tout le produit et non sur une seule partie selon l'art antérieur.

Le niveau de compression de l'article est de préférence entre 25 et 45 mmHg.

Les avantages ressortent bien de l'invention. L'identification inattendue d'un titrage spécifique de l'âme en élasthanne dans des valeurs très au-delà des besoins par rapport à la compression substantielle permet de concevoir un article textile de compression / contention se différenciant dimensionnellement des articles de mêmes caractéristiques en donnant des capacités optimisées pour l'enfilage de l'article sur des membres inférieurs ou supérieurs selon les applications.

Un autre avantage réside dans l'amélioration apportée sur le pied - là encore pour faciliter l'enfilage. La zone entre la pointe et le démarrage du cou-de-pied est encore plus assouplie, de façon à faciliter l'enfilage de la pointe et du début du pied et améliorer le confort au porter. Pour ce faire, un changement de trame est opéré dans cette zone : on passe pour l'exemple applicatif pour l'article selon l'invention avec une trame utilisée sur le reste de l'article (EA 940 dTex) à une trame "classique" avec une âme de 390dTex.

**Revendications**

1. Article de contention / compression ayant un niveau de compression élevé supérieur à 20mmHg du type tricoté comprenant un fil de trame guipé en double hélice avec une âme centrale en élastomère et notamment en élasthanne tricoté sur l'ensemble de l'article confectionné **caractérisé en ce qu'**il présente en combinaison les caractéristiques

   a) le titrage représentant la masse linéique du fil d'élasthanne contenu dans le fil de trame en dtex est compris entre 750 et 1.700 dTex,

   b) l'article présente des caractéristiques d'allongement dimensionnel sur toute sa surface mettant en oeuvre la formule :

$$\frac{Titre}{\sqrt{Comp.moy}} \geq K * \frac{Périm.app.\min}{L\arg.à.plat}$$

   où le facteur K est égal à 55 et exprimé en $\dfrac{dTex}{\sqrt{mmHg}}$

   c) l'article présente une déformation selon un coefficient d'allongement en situation et pour les dimensions minimales d'applications inférieur à 25%,

   d) la formule

$$\frac{Périm.app.\min}{L\arg.à.plat}$$

   représente la caractéristique dimensionnelle de l'invention,

   e) la formule

$$\frac{Titre}{\sqrt{Comp.moy}}$$

   représente le rapport entre le titrage du fil de trame guipé en élasthanne et le niveau de compression,

   f)

   → le Titre représente la masse linéique du fil d'élasthanne constituant l'âme contenue dans le fil de trame en dtex, et par extension de l'élastomère utilisé.

   → Com. Moy représente la compression moyenne dans la classe de compression concernée en mmHg selon la norme NF G30-102

   → Larg. à plat représente la largeur à plat du produit mesurée selon la nature de l'article, à la cheville en cm (selon la taille), la largeur au niveau du poignet pour un manchon positionné sur le membre supérieur.

   → Périm.app.min représente le périmètre d'application minimum selon la nature de l'article, à la cheville en cm (suivant la taille concernée) ou du poignet pour un manchon positionné sur le membre supérieur.

   g) et **en ce que** la combinaison des dites caractéristiques permet l'obtention pour une même classification un article de dimensions supérieures à un article comparable de même catégorie),

2. Article, selon la revendication 1, **caractérisé en ce que** l'âme en élastomère et élasthanne particulièrement présente un titrage de l'ordre de 900 à 1.000 dTex.

3. Article de compression, selon la revendication 1, **caractérisé en ce que** le niveau de compression est établi entre 25 et 45 mmHg.

**4.** Article, selon l'une quelconque des revendications 1 à 3, caractérisé dans son application aux bas de contention / compression.

**5.** Article, selon l'une quelconque des revendications 1 à 3, caractérisé dans son application aux manchons, coudières portés par les membres supérieurs de l'individu.


**Patentansprüche**

**1.** Stütz-/Kompressionsartikel mit einem hohen Kompressionsgrad von mehr als 20 mm Hg nach Art der gestrickten Produkte mit einem doppelspiralig umzwirnten Schussfaden mit einer zentralen Seele aus Elastomer, und insbesondere aus Elasthan, gestrickt über den gesamten Konfektionsartikel, **dadurch gekennzeichnet, dass** er in Kombination die nachstehenden Merkmale aufweist:

a) der Titer, der die lineare Dichte des im Schussfaden enthaltenen Elasthangarns in dtex ausdrückt, liegt zwischen 750 und 1700 dtex,
b) der Artikel weist auf der gesamten Oberfläche Maßdehnungseigenschaften auf nach der Formel:

$$\frac{Titer}{\sqrt{Durchschn.\ Komp.}} \geq K * \frac{Mind.anw.umfang}{Flachbreite}$$

wobei der Faktor K gleich 55 ist und ausgedrückt wird in $\dfrac{dTex}{\sqrt{mm\ Hg}}$

c) der Artikel weist eine Verformung gemäß einem situativen Dehnungskoeffizienten unter 25 % bei den minimalen Anwendungsmaßen auf,
d) die Formel

$$\frac{Mind.anw.umfang}{Flachbreite}$$

bezeichnet die Maßeigenschaft der Erfindung,
e) die Formel

$$\frac{Titer}{\sqrt{Durchschn.\ Komp.}}$$

bezeichnet das Verhältnis zwischen dem Titer des umzwimten Schussfadens aus Elasthan und der Kompressionsstufe,
f)

→ der *Titer* bezeichnet die in dtex ausgedrückte lineare Dichte des Elasthangarns, das die im Schussfaden enthaltene Seele bildet, und im weiteren Sinne des verwendeten Elastomers.
→ *Durchn. Komp.* bezeichnet die durchschnittliche Kompression in der betroffenen Kompressionsklasse in mm Hg nach der Norm NF G30-102.
→ *Flachbreite* bezeichnet die Flachbreite des Produkts, gemessen, je nach Art des Artikels, am Knöchel in cm (je nach Größe), bzw. die Breite im Bereich des Handgelenks bei einer Bandage auf der oberen Extremität.
→ *Mind.anw.umfang* bezeichnet den minimalen Anwendungsumfang je nach Art des Artikels, am Knöchel in cm (je nach der betroffenen Größe) bzw. am Handgelenk bei einer Bandage auf der oberen Extremität.

g) und **dadurch gekennzeichnet, dass** durch die Kombination der besagten Merkmale bei der gleichen Klassifikation ein Artikel mit größeren Abmessungen im Verhältnis zu einem vergleichbaren Artikel der gleichen Kategorie erzielt wird.

**2.** Artikel nach Anspruch 1, **dadurch gekennzeichnet, dass** die Seele aus Elastomer - und insbesondere aus Elasthan - einen Titer in der Größenordnung von 900 bis 1000 dtex aufweist.

**3.** Kompressionsartikel nach Anspruch 1, **dadurch gekennzeichnet, dass** die Kompressionsstufe zwischen 25 und 45 mm Hg liegt.

**4.** Artikel nach einem der Ansprüche 1 - 3, gekennzeichnet in seiner Anwendung auf Stütz-/Kompressionsstrümpfe.

**5.** Artikel nach einem der Ansprüche 1 - 3, gekennzeichnet in seiner Anwendung auf Armbandagen oder Ellbogenbandagen, die an den oberen Extremitäten der betroffenen Person getragen werden.

**Claims**

**1.** A compression article of the knitted type exhibiting a high level of compression greater than 20 mmHg comprising a double-wrapped weft yarn with a central core of elastomer and in particular of elastane knitted throughout the finished article, **characterised in that** it has the following features in combination:

a) the linear density representing the mass per unit length of the elastane yarn contained in the weft yarn in dtex is between 750 and 1700 dtex,
b) the article has characteristics of dimensional elongation over its entire surface in accordance with the formula:

$$\frac{Yarn\ count}{\sqrt{Avg.\ comp.}} \geq K * \frac{Min.\ app.\ perimeter}{Lay-flat\ width}$$

in which the factor K is equal to 55 and expressed in

$$\frac{dTex}{\sqrt{mmHg}}$$

c) the article exhibits deformation according to an *in situ* coefficient of elongation for minimum application dimensions of less than 25%,
d) the formula

$$\frac{Min.\ app.\ perimeter}{Lay-flat\ width}$$

represents the dimensional characteristic of the invention,
e) the formula

$$\frac{Yarn\ count}{\sqrt{Avg.\ comp.}}$$

represents the ratio between the linear density of the wrapped elastane weft yarn and the compression level,
f)

⇒ Yam count represents the mass per unit length of the elastane yarn contained in the weft yarn in dtex, and by extension of the elastomer used;
⇒ Avg. comp. represents the average compression in the compression class in question in mmHg to standard NF G30-102;
⇒ Lay-flat width represents the lay-flat width of the product measured depending on the nature of the article, at the ankle in cm (according to size), or the width at the level of the wrist for a sleeve positioned on the upper limb;
⇒ Min. app. perimeter represents the minimum application perimeter depending on the nature of the article, at the ankle in cm (according to the size in question) or of the wrist for a sleeve positioned on the upper limb.

g) and **in that** the combination of said features allows an article of greater dimensions than a comparable article of the same category to be obtained for the same classification.

2. An article according to claim 1, **characterised in that** the core of elastomer and in particular elastane exhibits a linear density of the order of 900 to 1000 dtex.

3. A compression article according to claim 1, **characterised in that** the level of compression is set at between 25 and 45 mmHg.

4. An article according to any one of claims 1 to 3, **characterised by** use thereof in compression stockings.

5. An article according to any one of claims 1 to 3, **characterised by** use thereof in sleeves or elbow braces worn on the upper limbs of an individual.

**Fig. 1**          **Fig. 2**          **Fig. 3**

**Fig. 4**

**Fig. 5**

**Fig. 6**

**Fig. 7**

**TABLEAU 5**

..........construction classique

_____construction selon l'invention

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2885035
- US 2006247566 A
- FR 2853525
- FR 2789301
- WO 0001332 A
- WO 0164152 A
- WO 2008053159 A
- FR 2804695
- FR 2888855
- FR 2896808
- US 20080171959 A

- FR 2703243
- FR 2852509
- EP 927014 A
- WO 2007113430 A
- FR 2654925
- WO 2004106607 A
- EP 1878822 A
- US 7441419 B
- US 7434423 B
- EP 1709947 A
- EP 1621164 A

**Littérature non-brevet citée dans la description**

- *Phlébologie,* 2009, vol. 62, 1